# EUROPEAN PATENT SPECIFICATION

(11) **EP 3 986 782 B1**
(45) Date of publication and mention of the grant of the patent: **30.07.2025**
(21) Application number: 20753404.1
(22) Date of filing: 15.06.2020
(51) Int. Cl.: B63H 16/04

(54) **PADDLE WITH IMPROVED EFFICIENCY**
PADDEL MIT VERBESSERTEM WIRKUNGSGRAD
PAGAIE À EFFICACITÉ AMÉLIORÉE

(30) Priority: 18.06.2019 HU 1900118 U
(43) Date of publication of application: 27.04.2022
(73) Proprietor: Bestpaddle Kft., 2610 Nötincs (HU)
(72) Inventor: GRÁF, Mihály, 2151 (HU)
(74) Representative: Ronaszéki, Tibor
(86) International application number: PCT/HU2020/000020
(87) International publication number: WO 2020/254843

(56) References cited:
- WO-A1-2018/029451
- RU-C1- 2 056 327
- RU-C1- 2 057 685

## Description

The object of the invention relates to a paddle with improved efficiency, especially a competition paddle for kayaks, which has a paddle shaft and a blade piece positioned at least at one of the ends of the paddle shaft, at least one long slot is arranged in the blade piece, and the cut-out has an inlet opening located on the pressure side of the blade piece, and an outlet opening located on the suction side of the blade piece, where the blade piece has a side protrusion starting from the inlet opening of the slot on the pressure side of the blade piece and progressing along a back surface of the suction side of the blade piece, separated from the suction side of the blade piece by a gap, and has an internal surface facing the pressure side of the blade piece, and so a flow channel is formed between the inlet opening and the outlet opening of the slot of the blade piece, enclosed by the overlap of the internal surface of the side protrusion facing the pressure side of the blade piece and the back surface of the suction side of the blade piece, furthermore each points of a delimiting edge of the inlet opening of the slot on the pressure side of the blade piece, and each points of a delimiting edge of the side protrusion bordering the outlet opening of the slot on the suction side of the blade piece which are falling in at least one cross-plane of the blade piece are separated from each other by a distance, furthermore the blade piece has a flow-influencing piece curved towards the pressure side of the blade piece along at least a part of an leading edge of the blade piece.

Water sports are a popular pastime all over the world. There are numerous versions of water vehicles "propelled" by various human forces. These include vessels and boats made to move with the use of paddles. Numerous solutions have become known for facilitating paddling and increasing the stability of the boat. Some of these solutions endeavour to achieve floating stability and fast movement of the vehicle with the design of the boat's hull. While another group of solutions aims at making paddling easier.

Publication document number US 2013/206052 discloses a boat hull that has a supplementary hull for accommodating the paddle. Using this makes it safer to get into and out of the boat. Also, it prevents the paddle from falling out of the boat during getting into and out of the boat. However, the disadvantage of the solution is that it does not make paddling easier nor is it easier to manoeuvre with the paddle.

Publication document number US 2008/0254691 discloses a solution the essence of which is that the length of the blade and its front face curve have been increased to increase the stability of the kayak paddle during paddling and the efficiency of thrusting. Also to reduce shaking the formation of long openings leading straight through the blade are proposed. The solution may well improve the efficiency of the thrust and reduce the shaking of the paddle during paddling.

Its disadvantage, however, is that the form of the blade does not facilitate the lifting of the blade out of the water. Although, especially in the case of competition blades, at the end of the paddling motion lifting the blade out of the water involves significant effort and represents a serious loss of time.

Another disadvantage is that the straight slots formed in the blade permitting free flow actually reduce the load that may be exerted on the blade.

Patent specifications with numbers RU 2.056.327, RU 2.057.685, and WO 2018/029451 are also known of. Each of these has slotting positioned substantially perpendicular to the longitudinal axis of the paddle and protrusions protruding out from the slotting. However, these solutions do not efficiently support the person using the paddle in being able to lift the paddle out of the water more easily and at greater speed following each stroke, as well as being able to transmit greater force to the medium via the paddle while performing a stroke.

The main idea of the patent specifications RU 2.056.327, is that this paddle is its multiple slots created perpendicularly of the paddle main axis. As can be seen in the cross section A-A picture of the specification, these slots are designed to promote the acceleration of the fluid which is flowing thorough the slot from the pressure side to the suction side. This kind of flow helps to maintain the boundary layer stability around the suction side of the blade i.e. improves its efficiency. However, for such flow to occur through the slots, the paddle must move perpendicular to the water surface, i.e. axially. This type of movement happens for only a very short time during a full stroke: when the paddle enters the water. As the time of this phase is very short, and also from an anatomical point of view, the kayaker cannot use it to exert effective force. Furthermore, this solution makes the last phase of the stroke, the exit, more difficult, as the flow is reversed and in this case the gaps impair the flow around the paddle. In summary, this solution will only provide a negligible increase in efficiency for kayakers.

Our aim with the solution according to the invention is to overcome the deficiencies of the known versions and elaborate a blade structure that facilitates the lifting of the blade from the water and simultaneously shortens the time required for this lifting, while making it possible for the blade to be subjected to a greater force.

The recognition that led to the blade structure according to the invention was that if deviating from that known of not only a simple slot is formed in the blade, but a uniquely formed pipe-like channel is created between the pressure side and the suction side of the blade, through which the water does not just simply pass during the movement of the blade, instead, differently to the conventional way, its direction of movement also changes to the chord direction of the blade, then as a result of the formation of the gap the flow around the blade improves, as a result of which greater upward force is created on the blade profile, and this increases upwards force and makes it possible to subject a greater force on the blade, and so the task may be solved.

In accordance with the set objective the paddle with improved efficiency according to the invention, particularly a competition paddle for kayaks, - which has a paddle shaft and a blade piece positioned at least at one of the ends of the paddle shaft, at least one long slot is arranged in the blade piece, and the slot has an inlet opening located on the pressure side of the blade piece, and an outlet opening located on the suction side of the blade piece, where the blade piece has a side protrusion starting from the inlet opening of the slot on the pressure side of the blade piece and progressing along a back surface of the suction side of the blade piece, separated from the suction side of the blade piece by a gap, and has an internal surface facing the pressure side of the blade piece, and so a flow channel is formed between the inlet opening and the outlet opening of the slot of the blade piece, enclosed by the overlap of the internal surface of the side protrusion facing the pressure side of the blade piece and the back surface of the suction side of the blade piece, furthermore each points of a delimiting edge of the inlet opening of the slot on the pressure side of the blade piece, and each points of a delimiting edge of the side protrusion bordering the outlet opening of the slot on the suction side of the blade piece which are falling in at least one cross-plane of the blade piece are separated from each other by a distance, furthermore the blade piece has a flow-influencing piece curved towards the pressure side of the blade piece along at least a part of an leading edge of the blade piece - is formed in such a way that the distance between those points of a delimiting edge of the inlet opening of the slot on the pressure side of the blade piece, and those points of a delimiting edge of the side protrusion bordering the outlet opening of the slot on the suction side of the blade piece which are falling in the same cross-plane, is situated in these cross-plane which perpendicular to the longitudinal main axis of the blade piece, furthermore the side protrusion of the blade piece starting from the inlet opening of the slot is located in the opposite direction to the flow-influencing piece behind the back surface of the suction side of the blade piece, and so the inlet opening of the slot is located between the flow-influencing piece of the blade piece and the outlet opening of the slot.

In the case of another embodiment of the paddle the distance formed by the overlap in a given cross-plane of the blade piece between the delimiting edge on the pressure side of the slot and the delimiting edge on the suction side of the slot is between 5 and 20 mm.

In the case of yet another different embodiment of the invention the gap between the internal surface of the side protrusion and the back surface of the suction side of the blade piece is between 2 and 10 mm. While the length of the slot of the blade piece is between 300 and 400 mm.

In the case of still another different embodiment of the paddle the internal surface of the side protrusion of the blade piece starting from the inlet opening of the slot is convex when viewed from the pressure side of the blade piece. Optionally the side protrusion of the blade piece starting from the inlet opening of the slot has at least a partially curved structure.

In the case of a further embodiment of the invention the part of the suction side of the blade piece located opposite the internal surface of the side protrusion starting from the inlet opening of the slot is convex when viewed from the pressure side of the blade piece. The part of the rear surface of the suction side of the blade piece located opposite the internal surface of the side protrusion starting from the inlet opening of the slot has at least a partially curved structure.

The paddle according to the invention has numerous preferred features. The most important of these is the as a consequence of the unique gap arrangement as compared to a conventional blade size the force that may be extracted from the blade according to the invention is greater in the case of a smaller blade.

A further advantage is that the upwards force exerted on the blade increases, and the component of this force pointing in the direction of movement "helps" the kayaker with paddling. If this force is determinant, then while paddling there are fewer detrimental movements, i.e. the paddle slips backwards while the competitor pushes forwards with it.

Then as a result of these features the kayaker using the paddle is able to make use of his or her strength with better efficiency during training or competitions. Also during hobby kayaking, in the case of the same degree of strength, it is possible to travel greater distances with the paddle according to the invention than in the case of conventional versions.

Further details of the paddle according to the invention will be explained by way of exemplary embodiments with reference to figures, wherein
Figure 1 shows a front view of a version of the paddle according to the invention,
Figure 2 shows a cross-section of the paddles according to figure 1 taken on the plane S-S.

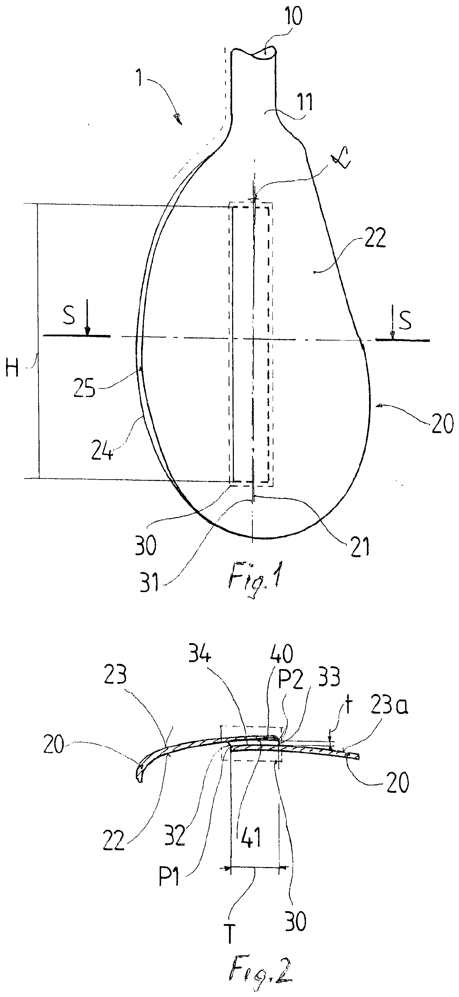

Figure 1 shows a detail of one end of the paddle according to the invention. It may be observed that the blade piece 20 of the paddle 1 is located at one end 11 of the paddle shaft 10, in the conventional way. Naturally the paddle shaft 10 may also have another blade piece 20, but as the other blade piece 20 has the same structure as that depicted in figure 1, this other blade piece 20 is not depicted.

The slot 30 may be easily observed on the pressure side 22 of the blade piece 20, the longitudinal axis 31 of which is at a minimal 2° angle "α" to the main axis 21 of the blade piece 20. It should be noted here that the main axis 21 of the blade piece 20 and the longitudinal axis 31 of the slot 30 may be coaxial, but a use is also conceivable where the angle "α" between the main axis 21 and the longitudinal axis 31 is even nearly 30°, for example. The value of this angle "α" is largely influenced by the length "H" of the slot 30, and by the values of the gap "t" and the distance "T" indicated in figure 2. Otherwise, the length "H" of the slot 30 is between 300 and 400 mm with consideration to the length of the blade 20 of an average competition paddle.

The flow-influencing piece 25 is located at the leading edge 24 of the blade piece 20, which runs substantially all the way along the leading edge 24 of the blade piece 20. The shape of the flow-influencing piece 25 may be seen well in figure 2. Figure 2 shows a cross-section of the blade piece 20 taken on the cross-plane "S". It may be easily seen that the flow-influencing piece 25 of the blade piece 20 is a curved edge bent towards the pressure side 22 of the blade piece 20, which viewed from the pressure side 22 of the blade piece 20 has a convex shape. The suction side 23 opposite the pressure side 22 of the blade piece 20 is delimited by the back surface 23a.

It is also easy to observe that the blade piece 20 has a side protrusion 40 starting from the inlet opening 32 of the slot 30 continuing along the blade piece 20. The side protrusion 40 is oriented in such a way that it progresses in the direction opposite to the flow-influencing piece 25 of the blade piece 20 along the back surface 23a of the suction side 23 of the blade piece 20.

The side protrusion 40 is positioned on the blade piece 20 in such a way that the internal surface 41 of the side protrusion 40 facing the pressure side 22 of the blade piece 20 and the back surface 23a of the suction side 23 on the other side of the blade piece 20 from the slot 30 opposite the flow-influencing piece 25 are next to each other and separated by a gap "t". The size of the gap "t" is between 2 and 10 mm, optionally 6 mm. Essentially, the internal space 41 of the side protrusion and the back surface 23a of the suction side 23 of the blade piece 20 in the vicinity of the side protrusion 40 surround the flow channel 34 of the slot 30.

Figure 2 clearly shows that the flow channel 34 of the slot 30 is delimited from the pressure side 22 of the blade piece 20 by the inlet opening 32 and by the outlet opening 33 on the suction side 23 of the blade piece 20. There is a 5 to 20 mm distance "T" between the inlet opening 32 and the outlet opening 33 spanning the overlapping part of the side protrusion 40 and the suction side 23 of the blade piece 20. The distance "T" is determined by projecting a straight line between the one reference point, on the left side of figure 2, which is the intersection of the delimiting edge "P1" delimiting the inlet opening 32 of the slot 30 on the pressure side 22 of the blade piece 20 and the cross-plane "S" perpendicular to the pressure side 22 of the blade piece, and the other reference point, which is the intersection of the outermost delimiting edge "P2" of the internal surface 41 of the side protrusion 40 and the same cross-plane "S".

Figure 2 also illustrates that the internal surface 41 of the side protrusion 40 starting from the slot 30 of the blade piece 20 is curved in a convex shape when viewed from the pressure side 22 of the blade piece 20. The same may be said about the back surface 23a of the suction side 23 of the blade piece 20 positioned opposite the internal surface 41 of the side protrusion 40 starting from the inlet opening 32 of the slot 30. This part of the back surface 23a viewed from the pressure side 22 is also curved in a convex shape.

It may be determined from the joint interpretation of figures 1 and 2 that the water does not only flow through the flow channel 34 of the slot 30 without changing direction. Here, as a consequence of the side protrusion 40 the water arriving from the inlet opening 32 of the slot 30 of the blade piece 20 changes direction in the flow channel 34 of the slot 30 and flows out through the outlet opening 32 of the slot 30 of the blade piece 20 sideways along the back surface 23a of the suction side 23 substantially in the direction of the cross-planes "S" perpendicular to the main axis 21 of the blade piece 20.

During the use of the paddle 1 according to the invention the blade piece 20 dipped into the water operates when the paddle 1 is pulled in such a way that a lower pressure is formed on the suction side 23 of the substantially aerofoil-shaped blade piece 20 than on the pressure side 22. In this way flow is created and the water flows from the inlet opening 32 in the pressure side 22 of the blade piece 20 into the flow channel 34 in the direction of the outlet opening 33. The free jet flowing through the outlet opening 33 of the slot 30 on the suction side 23 of the blade piece 20 refreshes the upper boundary layer. As a consequence of this the detachment sensitivity drops along the back surface 23a of the suction side 23 of the blade piece 20, and so the upwards force exerted on the blade piece 20 increases. Consequentially a greater pushing force may be gained from the paddle 1 with a smaller blade piece 20 movement, as during paddling the paddle 1 suffers fewer detrimental movements, i.e. slipping back while the person paddling "pushes forward" in the water by pushing the paddle 1.

The paddle according to the invention may be used to good effect to implement more efficient paddling, particularly in competition sports, as a kayak paddle.

**List of reference signs**

| | | | |
|---|---|---|---|
| 1 | paddle | | |
| 10 | paddle shaft | 11 | one end |
| 20 | blade piece | 21 | main axis |
| | | 22 | pressure side |
| | | 23 | suction side |
| | | 23a | back surface |
| | | 24 | leading edge |
| | | 25 | flow-influencing piece |
| 30 | slot | 31 | longitudinal axis |
| | | 32 | inlet opening |
| | | 33 | outlet opening |
| | | 34 | flow channel |
| 40 | side protrusion | 41 | internal surface |
| "H" | length | | |
| "P1" | delimiting edge (of the inlet opening 32) | | |
| "P2" | delimiting edge (of the outlet opening 33) | | |
| "S" | cross-plane | | |
| "t" | | | |
| "T" | distance | | |
| "α" | angle | | |

## Claims

1. Paddle with improved efficiency, particularly a competition paddle for kayaks which has a paddle shaft (10) and a blade piece (20) positioned at least at one of the ends (11) of the paddle shaft (10), at least one long slot (30) is arranged in the blade piece (20), and the slot (30) has an inlet opening (32) located on the pressure side (22) of the blade piece (20), and an outlet opening (33) located on the suction side (23) of the blade piece (20), where the blade piece (20) has a side protrusion (40) starting from the inlet opening (32) of the slot (30) on the pressure side (22) of the blade piece (20) and progressing along a back surface (23a) of the suction side (23) of the blade piece (20), separated from the suction side (23) of the blade piece (20) by a gap (t), and has an internal surface (41) facing the pressure side (22) of the blade piece (20), and so a flow channel (34) is formed between the inlet opening (32) and the outlet opening (33) of the slot (30) of the blade piece (20), enclosed by the overlap of the internal surface (41) of the side protrusion (40) facing the pressure side (22) of the blade piece (20) and the back surface (23a) of the suction side (23) of the blade piece (20), furthermore each points of a delimiting edge (P1) of the inlet opening (32) of the slot (30) on the pressure side (22) of the blade piece (20), and each points of a delimiting edge (P2) of the side protrusion (40) bordering the outlet opening (33) of the slot (30) on the suction side (23) of the blade piece (20) which are falling in at least one cross-plane of the blade piece (20) are separated from each other by a distance (T), furthermore the blade piece (20) has a flow-influencing piece (25) curved towards the pressure side (22) of the blade piece (20) along at least a part of an leading edge (24) of the blade piece (20), **characterised in that** the distance (T) between those points of a delimiting edge (P1) of the inlet opening (32) of the slot (30) on the pressure side (22) of the blade piece (20), and those points of a delimiting edge (P2) of the side protrusion (40) bordering the outlet opening (33) of the slot (30) on the suction side (23) of the blade piece (20) which are falling in the same cross-plane, is situated in these cross-planes which are perpendicular to the longitudinal main axis (21) of the blade piece (20), furthermore the side protrusion (40) of the blade piece (20) starting from the inlet opening (32) of the slot (30) is located in the opposite direction to the flow-influencing piece (25) behind the back surface (23a) of the suction side (23) of the blade piece (20), and so the inlet opening (32) of the slot (30) is located between the flow-influencing piece (25) of the blade piece (20) and the outlet opening (33) of the slot (30).

2. Paddle according to claim 1, **characterised by** that the distance (T) formed by the overlap in a given cross-plane of the blade piece (20) between the delimiting edge (P1) on the pressure side (22) of the slot (30) and the delimiting edge (P2) on the suction side (23) of the slot (30) is between 5 and 20 mm.

3. Paddle according to any of claims 1 or 2, **characterised by** that the gap (t) between the internal surface (41) of the side protrusion (40) and the back surface (23a) of the suction side (23) of the blade piece (20) is between 2 and 10 mm.

4. Paddle according to any of claims 1 to 3, **characterised by** that the length (H) of the slot (30) of the blade piece (20) is between 300 and 400 mm.

5. Paddle according to any of claims 1 to 4, **characterised by** that the internal surface (41) of the side protrusion (40) of the blade piece (20) starting from the inlet opening (32) of the slot (30) is convex when viewed from the pressure side (22) of the blade piece (20).

6. Paddle according to claim 5, **characterised by** that the side protrusion (40) of the blade piece (20) starting from the inlet opening (32) of the slot (30) has at least a partially curved structure.

7. Paddle according to any of claims 1 to 6, **characterised by** that the part of the suction side (23) of the blade piece (20) located opposite the internal surface (41) of the side protrusion (40) starting from the inlet opening (32) of the slot (30) is convex when viewed from the pressure side (22) of the blade piece (20).

8. Paddle according to claim 7, **characterised by** that the part of the rear surface (23a) of the suction side (23) of the blade piece (20) located opposite the internal surface (41) of the side protrusion (40) starting from the inlet opening (32) of the slot (30) has at least a partially curved structure.

## Patentansprüche

1. Paddel mit verbessertem Wirkungsgrad, insbesondere Wettkampfpaddel für Kajaks, das einen Paddelschaft (10) und ein zumindest an einem der Enden (11) des Paddelschafts (10) angeordnetes Schaufelblatt (20) aufweist, wobei in dem Schaufelblatt (20) zumindest ein langer Schlitz (30) angeordnet ist, und wobei der Schlitz (30) eine an der Druckseite (22) des Schaufelblatts (20) angeordnete Einlassöffnung (32) und eine an der Saugseite (23) des Schaufelblatts (20) angeordnete Auslassöffnung (33) aufweist, wobei das Schaufelblatt (20) einen Seitenvorsprung (40) aufweist, der von der Einlassöffnung (32) des Schlitzes (30) an der Druckseite (22) des Schaufelblatts (20) ausgeht und sich entlang einer von der Saugseite (23) des Schaufelblatts (20) durch einen Spalt (t) getrennten Rückfläche (23a) der Saugseite (23) des Schaufelblatts (20) fortsetzt, und eine der Druckseite (22) des Schaufelblatts (20) zugewandte Innenfläche (41) aufweist, und wobei so zwischen der Einlassöffnung (32) und der Auslassöffnung (33) des Schlitzes (30) des Schaufelblatts (20) ein Strömungskanal (34) gebildet ist, der von der Überlappung der der Druckseite (22) des Schaufelblatts (20) zugewandten Innenfläche (41) des Seitenvorsprungs (40) und der Rückfläche (23a) der Saugseite (23) des Schaufelblatts (20) umschlossen ist, wobei ferner jeder Punkt einer Begrenzungskante (P1) der Einlassöffnung (32) des Schlitzes (30) an der Druckseite (22) des Schaufelblatts (20) und jeder Punkt einer die Auslassöffnung (33) des Schlitzes (30) an der Saugseite (23) des Schaufelblatts (20) begrenzenden Begrenzungskante (P2) des Seitenvorsprungs (40), die in zumindest eine Querebene des Schaufelblatts (20) fallen, durch einen Abstand (T) voneinander getrennt sind, wobei ferner das Schaufelblatt (20) ein Strömungsbeeinflussungsstück (25) aufweist, das entlang zumindest eines Teils einer Vorderkante (24) des Schaufelblatts (20) zu der Druckseite (22) des Schaufelblatts (20) hin gekrümmt ist, **dadurch gekennzeichnet, dass** der Abstand (T) zwischen jenen Punkten einer Begrenzungskante (P1) der Einlassöffnung (32) des Schlitzes (30) an der Druckseite (22) des Schaufelblatts (20) und jenen Punkten einer die Auslassöffnung (33) des Schlitzes (30) an der Saugseite (23) des Schaufelblatts (20) begrenzenden Begrenzungskante (P2) des Seitenvorsprungs (40), die in dieselbe Querebene fallen, in diesen Querebenen liegt, die senkrecht zu der Längshauptachse (21) des Schaufelblatts (20) sind, wobei ferner der Seitenvorsprung (40) des Schaufelblatts (20), der von der Einlassöffnung (32) des Schlitzes (30) ausgeht, in der entgegengesetzten Richtung zu dem Strömungsbeeinflussungsstück (25) hinter der Rückfläche (23a) der Saugseite (23) des Schaufelblatts (20) angeordnet ist, und wobei so die Einlassöffnung (32) des Schlitzes (30) zwischen dem Strömungsbeeinflussungsstück (25) des Schaufelblatts (20) und der Auslassöffnung (33) des Schlitzes (30) angeordnet ist.

2. Paddel nach Anspruch 1, **dadurch gekennzeichnet, dass** der durch die Überlappung in einer gegebenen Querebene des Schaufelblatts (20) zwischen der Begrenzungskante (P1) an der Druckseite (22) des Schlitzes (30) und der Begrenzungskante (P2) an der Saugseite (23) des Schlitzes (30) gebildete Abstand (T) zwischen 5 und 20 mm beträgt.

3. Paddel nach einem der Ansprüche 1 oder 2, **dadurch gekennzeichnet, dass** der Spalt (t) zwischen der Innenfläche (41) des Seitenvorsprungs (40) und der Rückfläche (23a) der Saugseite (23) des Schaufelblatts (20) zwischen 2 und 10 mm beträgt.

4. Paddel nach einem der Ansprüche 1 bis 3, **dadurch gekennzeichnet, dass** die Länge (H) des Schlitzes (30) des Schaufelblatts (20) zwischen 300 und 400 mm beträgt.

5. Paddel nach einem der Ansprüche 1 bis 4, **dadurch gekennzeichnet, dass** die Innenfläche (41) des Seitenvorsprungs (40) des Schaufelblatts (20), die von der Einlassöffnung (32) des Schlitzes (30) ausgeht, von der Druckseite (22) des Schaufelblatts (20) aus gesehen konvex ist.

6. Paddel nach Anspruch 5, **dadurch gekennzeichnet, dass** der Seitenvorsprung (40) des Schaufelblatts (20), der von der Einlassöffnung (32) des Schlitzes (30) ausgeht, zumindest eine teilweise gekrümmte Struktur aufweist.

7. Paddel nach einem der Ansprüche 1 bis 6, **dadurch gekennzeichnet, dass** der Teil der Saugseite (23) des Schaufelblatts (20), der der Innenfläche (41) des Seitenvorsprungs (40), die von der Einlassöffnung (32) des Schlitzes (30) ausgeht, gegenüberliegt, von der Druckseite (22) des Schaufelblatts (20) aus gesehen konvex ist.

8. Paddel nach Anspruch 7, **dadurch gekennzeichnet, dass** der Teil der Rückfläche (23a) der Saugseite (23) des Schaufelblatts (20), der der Innenfläche (41) des Seitenvorsprungs (40), die von der Einlassöffnung (32) des Schlitzes (30) ausgeht, gegenüberliegt, zumindest eine teilweise gekrümmte Struktur aufweist.

## Revendications

1. Pagaie à efficacité améliorée, particulièrement une pagaie de compétition pour kayaks qui comporte un manche de pagaie (10) et un élément pale (20) positionné au moins à une des extrémités (11) du manche de pagaie (10), au moins une longue fente (30) est ménagée dans l'élément pale (20), et la fente (30) comporte une ouverture d'entrée (32) située du côté pression (22) de l'élément pale (20), et une ouverture de sortie (33) située du côté aspiration (23) de l'élément pale (20), dans laquelle l'élément pale (20) comporte une saillie latérale (40) démarrant à partir de l'ouverture d'entrée (32) de la fente (30) sur le côté pression (22) de l'élément pale (20) et progressant le long d'une surface arrière (23a) du côté aspiration (23) de l'élément pale (20), séparée du côté aspiration (23) de l'élément pale (20) par un espace (t), et comporte une surface interne (41) faisant face au côté pression (22) de l'élément pale (20), et ainsi un canal d'écoulement (34) est formé entre l'ouverture d'entrée (32) et l'ouverture de sortie (33) de la fente (30) de l'élément pale (20), délimité par le chevauchement de la surface interne (41) de la saillie latérale (40) faisant face au côté pression (22) de l'élément pale (20) et de la surface arrière (23a) du côté aspiration (23) de l'élément pale (20), en outre, chacun des points d'un bord de délimitation (P1) de l'ouverture d'entrée (32) de la fente (30) sur le côté pression (22) de l'élément pale (20) et chacun des points d'un bord de délimitation (P2) de la saillie latérale (40) bordant l'ouverture de sortie (33) de la fente (30) sur le côté aspiration (23) de l'élément pale (20) qui s'inscrivent dans au moins un plan transversal de l'élément pale (20) sont séparés les uns des autres d'une distance (T), en outre l'élément pale (20) comporte un élément influençant l'écoulement (25) courbé vers le côté pression (22) de l'élément pale (20) le long d'au moins une partie d'un bord d'attaque (24) de l'élément pale (20), **caractérisée en ce que** la distance (T) entre les points d'un bord de délimitation (P1) de l'ouverture d'entrée (32) de la fente (30) sur le côté pression (22) de l'élément pale (20), et les points d'un bord de délimitation (P2) de la saillie latérale (40) bordant l'ouverture d'entrée (33) de la fente (30) sur le côté aspiration (23) de l'élément pale (20) qui s'inscrivent dans le même plan transversal, est située dans ces plans transversaux qui sont perpendiculaires à l'axe principal longitudinal (21) de l'élément pale (20), en outre la saillie latérale (40) de l'élément pale (20) démarrant à partir de l'ouverture d'entrée (32) de la fente (30) est située dans la direction opposée à l'élément influençant l'écoulement (25) derrière la surface arrière (23a) du côté aspiration (23) de l'élément pale (20), et ainsi l'ouverture d'entrée (32) de la fente (30) est située entre l'élément influençant l'écoulement (25) de l'élément pale (20) et l'ouverture de sortie (33) de la fente (30).

2. Pagaie selon la revendication 1, **caractérisée en ce que** la distance (T) formée par le chevauchement dans un plan transversal donné de l'élément pale (20) entre le bord de délimitation (P1) sur le côté pression (22) de la fente (30) et le bord de délimitation (P2) sur le côté aspiration (23) de la fente (30) est comprise entre 5 et 20 mm.

3. Pagaie selon l'une ou l'autre des revendications 1 et 2, **caractérisée en ce que** l'écartement (t) entre la surface interne (41) de la saillie latérale (40) et la surface arrière (23a) du côté aspiration (23) de l'élément pale (20) est compris entre 2 et 10 mm.

4. Pagaie selon l'une quelconque des revendications 1 à 3, **caractérisée en ce que** la longueur (H) de la fente (30) de l'élément pale (20) est comprise entre 300 et 400 mm.

5. Pagaie selon l'une quelconque des revendications 1 à 4, **caractérisée en ce que** la surface interne (41) de la saillie latérale (40) de l'élément pale (20) démarrant à partir de l'ouverture d'entrée (32) de la fente (30) est convexe lorsqu'observée depuis le côté pression (22) de l'élément pale (20).

6. Pagaie selon la revendication 5, **caractérisée en ce que** la saillie latérale (40) de l'élément pale (20) démarrant à partir de l'ouverture d'entrée (32) de la fente (30) a une structure au moins partiellement courbée.

7. Pagaie selon l'une quelconque des revendications 1 à 6, **caractérisée en ce que** la partie du côté aspiration (23) de l'élément pale (20) située opposée à la surface interne (41) de la saillie latérale (40) démarrant à partir de l'ouverture d'entrée (32) de la fente (30) est convexe lorsqu'observée depuis le côté pression (22) de l'élément pale (20).

8. Pagaie selon la revendication 7, **caractérisée en ce que** la partie de la surface arrière (23a) du côté aspiration (23) de l'élément pale (20) située opposée à la surface interne (41) de la saillie latérale (40) démarrant à partir de l'ouverture d'entrée (32) de la fente (30) a une structure au moins partiellement courbée.
